# EUROPÄISCHE PATENTSCHRIFT

(11) **EP 1 093 777 B1**
(45) Veröffentlichungstag und Bekanntmachung des Hinweises auf die Patenterteilung: **05.10.2005**
(21) Anmeldenummer: 00122843.6
(22) Anmeldetag: 20.10.2000
(51) Int. Cl.: A61F 2/40

(54) **Schulter-Endoprothese**
Shoulder-endoprosthesis
Endoprothèse d'épaule

(30) Priorität: 22.10.1999 DE 29918669 U
(43) Veröffentlichungstag der Anmeldung: 25.04.2001
(73) Patentinhaber: Mathys AG Bettlach, 2544 Bettlach (CH)
(72) Erfinder: Mühlhäusler, Bernd, Dr., 06507 Bad Suderode (DE); Wahl, Diethard, Dr., 15537 Gosen (DE); Glien, Wilfried, Dr., 07639 Bad Klosterlausnitz (DE); Salomon, Dirk, 04626 Jonaswalde (DE)
(74) Vertreter: Körfer, Thomas

(56) Entgegenhaltungen:
- WO-A-98/08468
- WO-A-99/34756
- WO-A-99/37254
- DE-A- 3 605 630
- DE-A- 4 031 520
- FR-A- 2 666 221
- FR-A- 2 735 972
- US-A- 5 507 817

## Beschreibung

Die Erfindung betrifft eine Schulter-Endoprothese mit einem Gelenkkopf, mit einem Mittelteil, an dem der Gelenkkopf befestigbar ist, und mit einem Schaftteil, das ebenfalls an dem Mittelteil befestigbar ist.

### 1. Stand der Technik

Der Ersatz erkrankter oder durch Unfall geschädigter Gelenke gehört seit vielen Jahren zur täglichen Praxis in der Unfallchirurgie und Orthopädie. Insbesondere künstliche Hüft- und Kniegelenke haben sich einen festen Platz in der Medizin erobert.

Innerhalb der letzten Jahre wird auch der Ersatz geschädigter Schultergelenke immer häufiger mit Erfolg angewendet.

Die Anforderungen an künstliche Gelenke ergeben sich aus ihrer mechanischen Belastung, die als Wechsellast auftritt und über viele Jahre ertragen werden muß, sowie aus den anatomischen Gegebenheiten, an die sich das künstliche Gelenk anpassen muß, sowie aus den Milieueinflüssen, denen die verwendeten Werkstoffe permanent widerstehen müssen.

Für den Bau künstlicher Gelenke werden heute grundsätzlich drei Werkstoffkategorien eingesetzt. Dies sind korrosionsfeste, biotolerante Metalle; biokompatible Keramiken und Kunststoffe, die den jeweiligen speziellen mechanischen Ansprüchen genügen und deren Alterungsverhalten die Funktionstüchtigkeit des Gelenkes über viele Jahre garantiert.

Spezielle Probleme, die sich für die gesamte Gelenkendoprothetik ergeben, sind die jeweiligen individuellen anatomischen Verhältnisse, an die das künstliche Gelenk angepasst werden muß, um die im gesunden Gelenk vorhandenen biomechanischen und kinematischen Verhältnisse im künstlichen Gelenk zumindest annähernd wiederherzustellen.

Je nach den individuellen Bedingungen werden deshalb verschiedene Größen und Konstruktionen von künstlichen Gelenken hergestellt, um möglichst jedem Anwendungsfall gerecht werden zu können. Relativ einfach ist dies möglich bei Hüftgelenken, da das Hüftgelenk ein Kugelgelenk ist und infolgedessen anatomisch, biomechanisch und kinematisch verhältnismäßig leicht überschaubar ist.

Wesentlich komplizierter sind die Verhältnisse im Kniegelenk, da die Freiheitsgrade des Kniegelenks durch den Kapsel- und Bandapparat deutlich eingeschränkt sind und sich im natürlichen Gelenk Gleit- und Rollbewegung überlagern.

Auch im Schultergelenk sind die Verhältnisse relativ kompliziert, da das Schultergelenk einen außerordentlich großen Bewegungsumfang garantiert und die Gelenkpfanne (Glenoid) nur einen geringen Teil des Gelenkkopfes überdeckt.

Besonders schwierig werden die Verhältnisse im Falle des durch Unfall zerstörten Gelenkes, da jeder Einzelfall eine für sich besondere Problematik darstellt. Aus diesem Grunde werden Schulterprothesen in der Regel modular aufgebaut.

Es ergeben sich damit viele Kombinationsmöglichkeiten, die dem Individualfall relativ gut angepasst werden können.

Diese Modularität birgt jedoch auch Risiken in sich, die vor allem in verminderter Dauerfestigkeit bestehen können oder auch komplizierte Verbindungsmechanismen bedingen, die die Operationstechnik komplizieren und damit auch die Operationszeiten verlängern.

Aus DE-A-195 48 154 ist eine Schulter-Endoprothese mit einem Schaft, einem Kopfhalsteil und einer Kopfkalotte bekannt. Der Kopfhalsteil ist gegenüber dem Schaft verstellbar, so daß die effektive Länge des Schaftes verändert werden kann.

Aus EP-A 0 679 375 ist eine Schulter-Endoprothese bekannt, bei der das Mittelteil gegenüber dem Schaftteil in seiner Drehlage um die Schaftachse einstellbar ist. Das Mittelteil ist nicht längs der Schaftachse einstellbar und die notwendige Länge der Prothese wird durch unterschiedlich lange Mittelteile erzielt.

Auch bei der Schulter-Endoprothese, die aus FR-A 2 664 809 bekannt ist, erfolgt eine Längenanpassung ebenfalls durch verschieden lange Zwischenstücke.

In EP-B1-0 278 807 ist eine Schulterprothese beschrieben, die aus einem Oberarmstück und einem die Gelenkkugel tragenden Stück besteht. Beide Teile sind in Längsrichtung gegeneinander verschiebbar, so dass anatomische Größenverhältnisse eingestellt werden können. Die Rotation der beiden Teile gegeneinander ist jedoch nicht fixiert. Auch anatomische Winkelverhältnisse des Kugelkopfes zum Humerusschaft können nicht eingestellt werden.

Aus DE-T2-38 76 087 ist eine Schulterprothese bekannt, die aus einem Schaftteil und einem Kopfteil besteht, die über eine Kegelsteckverbindung miteinander verbunden werden. Durch Trauma entstandene, sehr unterschiedliche anatomische Bedingungen können mit dieser Prothese weder im Kopfbereich noch im Schaftbereich berücksichtigt werden. Auch die Winkelverhältnisse sind nicht einstellbar.

Aus EP-B1-0 127 503 ist eine Schulterprothese bekannt, die aus einem Humerusschaft und einer Gelenkpfanne besteht. Kopf und Schaft sind bei dieser Prothese im Vergleich zum natürlichen Schultergelenk vertauscht, so dass das Kopfteil am Schulterblatt befestigt wird. Vorteil der Konstruktion ist die Luxationssicherheit in allen anatomischen Lagen. Dieses Gelenk ist jedoch nur begrenzt anwendbar, da es insbesondere bei traumatischen Indikationen den verschiedenen Bedingungen nicht angepasst werden kann. Das Glenoidteil ist außerdem sehr voluminös und infolgedessen nicht in jedem Falle anatomisch günstig zu implantieren.

Aus DE-A-4 031 520 ist eine Schulter-Endoprothese gemäß dem Oberbegriff von Anspruch 1 bekannt.

### 2. Zusammenfassung der Erfindung

Das Ziel der Erfindung besteht in einer modular aufgebauten Schulterprothese, die sehr unterschiedlichen anatomischen Gegebenheiten, insbesondere auch traumatischen Indikationen, gerecht wird.

Erfindungsgemäß wird dieses Ziel dadurch erreicht, daß das Mittelteil gegenüber dem Schaftteil längs dessen Schaftachse einstellbar ist und daß ferner das Mittelteil gegenüber dem Schaftteil auch in seiner Drehlage um die Schaftachse einstellbar ist.

Vorzugsweise weist das Mittelteil eine Bohrung auf und ist das Schaftteil mittels eines Spannelements in dieser Bohrung festspannbar.

Vorzugsweise sind das proximale Ende des Schaftteils und das Spannelement mit zusammenwirkenden konischen Flächen versehen und wird das Schaftteil durch Konusklemmung an dem Mittelteil festgespannt.

Das proximale Ende des Schaftteils kann als konischer Zapfen ausgebildet sein und das Spannelelement kann eine spreizbare Hülse mit konischen Innenfläche sein. Durch Spannen der Hülse auf den konischen Zapfen wird die Hülse aufgeweitet, so daß sie in der Bohrung des Mittelteils fixiert wird.

Eine andere Möglichkeit besteht darin, das proximale Ende des Schaftes mit einer konischen Bohrung zu versehen und spreizbar auszubilden und als Spannelement einen konischen Zapfen in diese Bohrung zu spannen, so daß sich das aufgeweitete Ende des Schaftteils in der Bohrung des Mittelteils fixiert.

Durch Positionieren des proximalen Endes des Schaftteils zusammen mit dem noch lose darauf sitzenden Spannelement innerhalb der Bohrung des Mittelteils sind die Gesamtlänge der Prothese und der Rotationswinkel zwischen Mittelteil und Schaftteil einstellbar.

Vorzugsweise erfolgt die Einstellung der Gesamtlänge der Prothese stufenlos oder in Stufen kleiner 5 mm und die Einstellung des Rotationswinkels zwischen Mittelteil und Schaftteil ebenfalls stufenlos.

Vorzugsweise verfügt das Mittelteil über ein Element zur Befestigung von Knochenbruchstücken. Hierbei kann es sich um Krallen oder Spitzen zur Fixation von Knochenbruchstücken handeln.

Nach dem Grundgedanken der Erfindung ist die Schulter-Endoprothese modular aufgebaut und besteht sie aus dem Schaft, dem Spannelement, dem Mittelteil und dem Gelenkkopf. Die Verbindung von Schaft und Mittelteil ist stufenlos oder in kleinen Stufen in axialer Richtung der Schaftachse verstellbar. Die Verbindung von Schaft und Mittelteil ist ferner stufenlos in ihrem Rotationswinkel um die Schaftachse (Ante- und Retroversionswinkel) verstellbar. Die Verbindung von Schaft und Mittelteil ist in der Lage Rotationsmomente aufzunehmen. Die Verstellung des Implantates längs der Schaftachse und die Rotation um sie erfolgen intraoperativ. Diese Verstellungen des Implantats erfolgen dabei am implantierten Schaft.

Einerseits können die Teile der Schulterprothese somit durch einfache und sichere Elemente verbunden werden, die auch während der Operation noch ausgetauscht werden können, und anderseits können die Winkel- und Längenverhältnisse stufenlos eingestellt werden.

Durch den modularen Aufbau sind Schäfte in verschiedenen Längen und Durchmessern einsetzbar, sind solide und modulare Köpfe mit verschiedenen Außengeometrien einsetzbar und können die Mittelteile mit verschiedenen Außengeometrien verwendet werden. Der Kopf kann auch einteilig mit dem Mittelteil sein.

Das Schaftteil und das Mittelteil können aus Titan- oder Kobalt-Chrom-Legierungen und der Gelenkkopf kann aus einer Kobalt-Chrom-Legierung oder Keramik hergestellt sein.

### 3. Beschreibung der Erfindung

Ausführungsbeispiele der Erfindung werden nachfolgend anhand der Zeichnung näher beschrieben. Es zeigen:
- Fig. 1: den prinzipiellen Aufbau und die Funktion der Schulter-Endoprothese;
- Fig. 2: in einer geschnittenen Seitenansicht die Schulter-Endoprothese ohne aufgesetzter Kugel mit konischem Spannelement im Schaft und formschlüssiger Verbindung zum Mittelteil;
- Fig. 3: in einer geschnittenen Seitenansicht die Schulter-Endoprothese ohne aufgesetzte Kugel mit einem hülsenförmigen Spannelement und einer Sicherungsschraube und formschlüssiger Verbindung zum Mittelteil;
- Fig. 4: in einer geschnittenen Seitenansicht das Implantat analog Fig. 2 jedoch mit kraftschlüssiger Verbindung zum Mittelteil;
- Fig. 5: in einergeschnittenen Seitenansicht das Implantat analog Fig. 3 jedoch mit kraftschlüssiger Verbindung zum Mittelteil und
- Fig. 6: ein Detail zur Befestigung von Knochensubstanz.

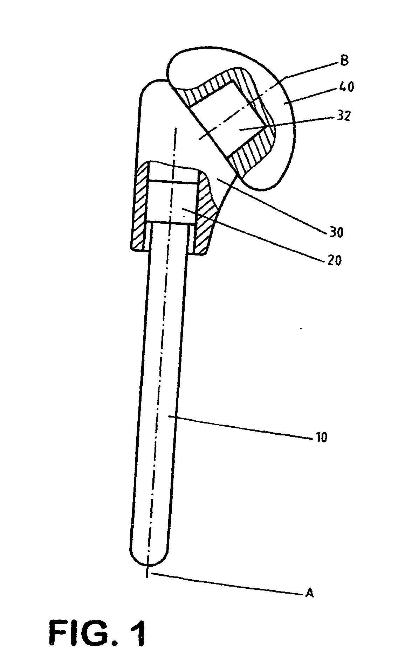

Die Schulter-Endoprothese in **Fig. 1** weist einen Schaft **10**, ein darauf aufgesetztes Spannelement **20**, ein darauf aufgesetztes und gegenüber der Schaftachse **A** um 360° drehbares und entlang der Schaftachse **A** in der Höhe verstellbares Mittelteil **30** auf. Dieses besitzt eine Aufnahme **32**, deren Achse **B** windschief oder winklig zur Schaftachse **A** angeordnet ist. Die Aufnahme **32** dient der Befestigung eines Kopfes **40** der Schulter-Endoprothese.

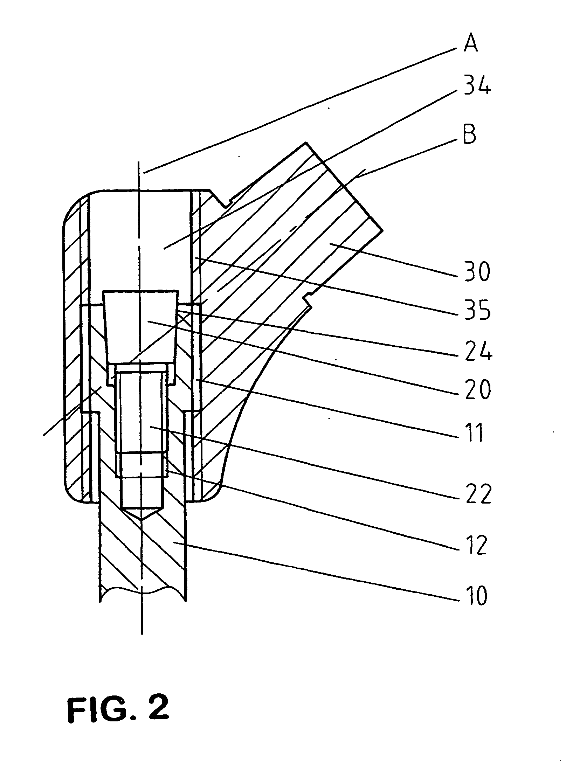

Bei dem Ausführungsbeispiel von **Fig. 2** befindet sich am proximalen Ende des Schaftes **10** ein dehnbares oder spreizbares Außengewinde **11** und eine Gewindebohrung **12**. Das Mittelteil **30** hat eine durchgehende Bohrung **34** mit innenliegendem Gewinde **35**. Auf das Außengewinde **11** des Schaftes **10** ist das Mittelteil **30** mit seinem innenliegenden Gewinde **35** entlang der Achse **A** aufgeschraubt. Das Spannelement **20** wird durch einen Gewindezapfen **22** und einen sich daran anschließenden Konus **24** gebildet. Durch Einschrauben des Gewindezapfens **22** des Spannelementes **20** in die im proximalen Teil des Schaftes **10** eingebrachte Gewindebohrung **12** erfolgt über den Konus **24** die Fixierung des Schaftes **10** am Mittelteil **30**. Die effektive Länge des Schaftes **10** kann durch unterschiedlich weites Einschrauben des Schaftes **10** in das Mittelteil **30** verändert und an die Erfordernisse des jeweiligen Falles angepaßt werden. Nach dem Einstellen der Schaftlänge wird das Spannelement **20** eingeschraubt und dadurch der Schaft **10** am Mittelteil **30** festgeklemmt. Durch Herausdrehen des Spannelements **20** kann die Klemmung auch während einer Operation wieder gelöst werden, um z.B. die Schaftlänge oder die Drehlage des Schaftes **10** zu korrigieren.

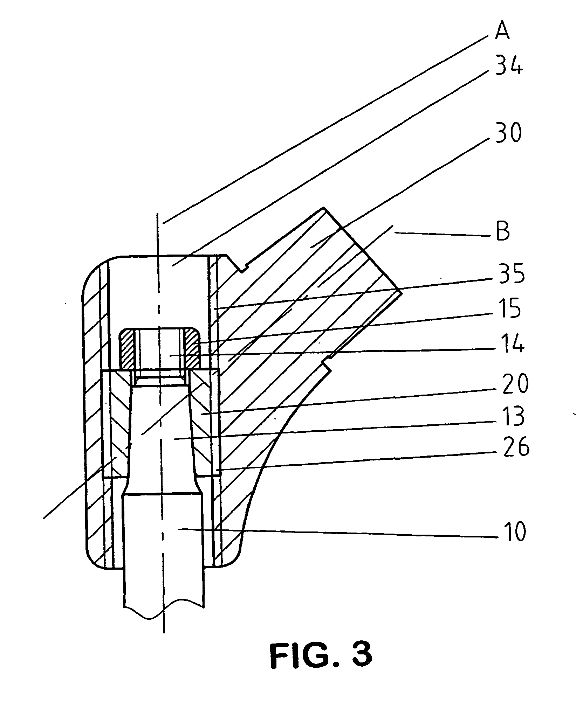

Bei dem Ausführungsbeispiel von **Fig. 3** ist das proximale Ende des Schaftes **10** als ein konischer Zapfen **13** mit darüberliegendem Gewindezapfen **14** ausgebildet. Das Spannelement **20** ist eine spreizbare Hülse mit einem sich in distaler Richtung konisch erweiternden Innendurchmesser und mit einem Außengewinde **26**. Das hülsenförmige Spannelement **20** wird auf den konischen Zapfen **13** aufgesetzt und mit einer Mutter **15** gesichert und dann mit seinem Außengewinde **26** entlang der Achse **A** in das Mittelteil **30** eingeschraubt, bis der Schaft **10** die gewünschte effektive Länge hat. Durch Anziehen der Mutter **15** wird das hülsenförmige Spannelement **20** gegen den konischen Zapfen **13** gedrückt und dadurch radial aufgeweitet, was zu einer Verspannung in der Bohrung **34** des Mittelteils **30** und damit zu einer Fixierung der Länge des Schaftes **10** führt. Nach Lösen der Mutter **15** ist eine Korrektur der Schaftlänge möglich.

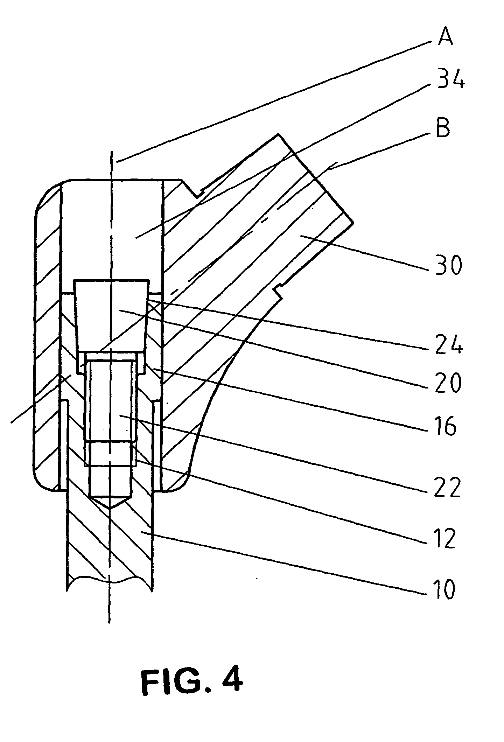

Das Ausführungsbeispiel von **Fig. 4** unterscheidet sich von dem nach **Fig. 2** dadurch, daß die durchgehende Bohrung **34** des Mittelteils **30** eine glatte Innenwand statt eines Gewindes hat. Die Schulter-Endoprothese nach **Fig. 4** besteht somit aus dem Schaft **10** an dessen proximalen Ende sich ein dehnbares oder spreizbares Endstück **16** mit glatter Außenfläche befindet. In dem Endstück **16** befindet sich wiederum die Gewindebohrung **12**. Das Mittelteil **30** wird mit seiner innenliegenden Bohrung **34** längs der Achse **A** auf das Endstück **16** des Schaftes **10** aufgesteckt. Durch Einschrauben des Gewindezapfens **22** des Spannelementes **20** in die Gewindebohrung **12** erfolgt über den Konus **24** die Fixierung des Schaftes **10** im Mittelteil **30**.

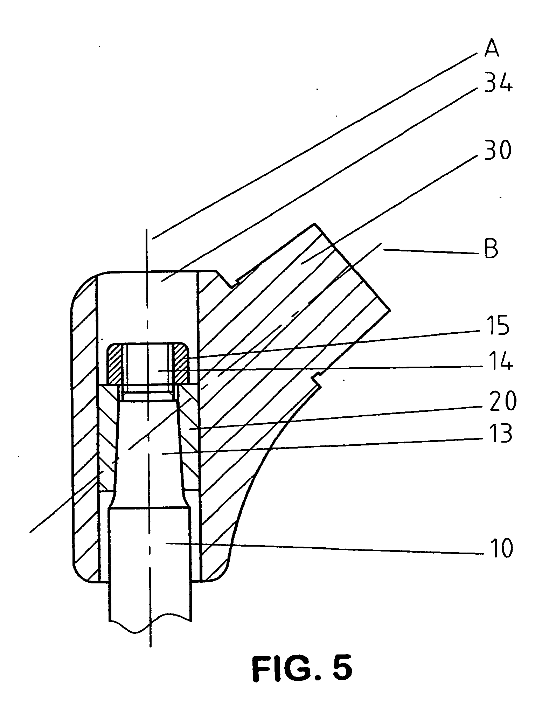

Das Ausführungsbeispiel von **Fig. 5** unterscheidet sich von dem nach **Fig. 3** ebenfalls nur dadurch, daß die durchgehende Bohrung **34** des Mittelteils **30** eine glatte Innenwand statt eines Gewindes hat. Bei dem Ausführungsbeispiel von **Fig. 5** wird daher das hülsenförmige Spannelement **20** auf den konischen Zapfen **13** aufgesetzt und wird die Mutter **15** aufgeschraubt. Das Mittelteil **30** wird dann mit seiner innenliegenden Bohrung **34** entlang der Achse **A** aufgesteckt. Durch Anziehen der Mutter **15** wird das Spannelement **20** auf den konischen Zapfen **13** gedrückt und dadurch radial aufgeweitet, was zu einer Verspannung in der Bohrung **34** des Mittelteils **30** führt.

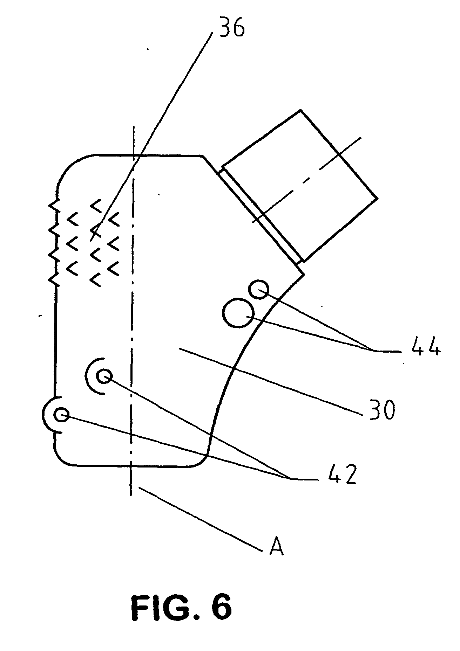

**Fig. 6** zeigt die Ausführung des Mittelteiles **30** mit spitzenartigen Erhebungen **36**. Diese sind proximal-lateral, reihenförmig, spaltenförmig, diagonal oder in beliebiger Form so angeordnet, daß Knochenbruchstücke durch Aufdrücken auf diese spitzenartigen Erhebungen gegenüber Zugkräften entlang der Achse **A** formschlüssig fixiert werden können. Am distalen Ende des Mittelteiles der Prothese sind Bohrungen **42** angebracht, die ein Herunterziehen und primäres Halten der Knochenbruchstücke über die spitzenartigen Erhebungen **36** ermöglichen. Medial ausgerichteten Bohrungen **44** dienen der Aufnahme von Fäden, die über die proximal-lateral aufgedruckten Knochenbruchstücke verlaufen und deren Abheben von den spitzenartigen Erhebungen **37** verhindern. Des weiteren sind die spitzenartigen Erhebungen **37** so gestaltet, daß die Oberfläche der Schulter-Endoprothese und damit auch die Mikrationsfläche der Knochenbruchstücke vergrößert wird.

### Bezugszeichenliste

- A: Schaftachse
- B: Achse der Aufnahme
- 10: Schaft
- 11: Aussengewinde
- 12: Gewindebohrung
- 13: konischer Zapfen
- 14: Gewindezapfen
- 15: Mutter
- 16: Endstück
- 20: Spannelement
- 22: Gewindezapfen
- 24: Konus
- 26: Aussengewinde
- 30: Mittelteil
- 32: Aufnahme
- 34: Bohrung
- 36: Erhebungen
- 40: Gelenkkopf
- 42: Bohrung (am distalen Ende)
- 44: (medial ausgerichtete) Bohrung

## Patentansprüche

1. Schulter-Endoprothese mit einem Gelenkkopf (40), mit einem Mittelteil (30), an dem der Gelenkkopf (40) befestigbar ist, und mit einem Schaftteil (10), das ebenfalls an dem Mittelteil (30) befestigbar ist, wobei das Mittelteil (30) gegenüber dem Schaftteil (10) längs dessen Schaftachse (A) einstellbar ist, wobei das Mittelteil (30) gegenüber dem Schaftteil (10) in seiner Drehlage um die Schaftachse (A) einstellbar ist, und das Mittelteil (30) eine Bohrung (34) aufweist,
**dadurch gekennzeichnet,**
**daß** das Mittelteil (30) mit seiner innenliegenden Bohrung (34) auf ein Endstück (16) des Schaftteils (10) aufsteckbar ist und das Schaftteil (10) mittels eines Spannelements (20) in dieser Bohrung (34) festspannbar ist.

2. Schulter-Endoprothese nach Anspruch 1, **dadurch gekennzeichnet, daß** das Schaftteil (**10**) durch eine Konusklemmung in der Bohrung (**34**) des Mittelteils (**30**) festspannbar ist.

3. Schulter-Endoprothese nach Ansptruch 2, **dadurch gekennzeichnet, daß** das proximale Ende (**16**) des Schaftteils (**10**) spreizbar ist und eine konische Bohrung (**12**) aufweist, in der das Spannelement (**20**) angreift.

4. Schulter-Endoprothese nach Anspruch 2, **dadurch gekennzeichnet, daß** das Spannelement (**20**) eine spreizbare Hülse mit einer konischen Innenfläche ist und das proximale Ende des Schaftteils (**10**) eine konische Außenfläche (**13**) aufweist, an der das Spannelement (**20**) angreift.

5. Schulter-Endoprothese nach einem der Ansprüche 1 bis 4, **dadurch gekennzeichnet, daß** die Bohrung (**34**) des Mittelteils (**30**) ein Innengewinde (**35**) aufweist und das Spannelement (**20**) bzw. das proximale Ende des Schaftteils (**10**) ein entsprechendes Außengewinde hat.

6. Schulter-Endoprothese nach einem der Ansprüche 1 bis 4, **dadurch gekennzeichnet, daß** die Bohrung (**34**) des Mittelteils (**30**) eine glatte Innenfläche hat und das Spannelement (**20**) bzw. das proximale Ende des Schaftteils (**10**) eine glatte Außenfläche hat.

7. Schulter-Endoprothese nach einem der Ansprüche 1 bis 6, **dadurch gekennzeichnet, daß** das Mittelteil (**30**) auf der Außenfläche über ein Element (**36**) zur Befestigung von Knochenbruchstücken verfügt.

## Claims

1. A shoulder endoprosthesis having a joint head (40), having a middle portion (30), to which the joint head (40) may be attached, and having a stem portion (10), which may likewise be attached to the middle portion (30), the middle portion (30) being adjustable relative to the stem portion (10) along the stem axis (A) thereof, the middle portion (30) being adjustable relative to the stem portion (10) in its rotational position about the stem axis (A), and the middle portion (30) comprising a bore (34), **characterised in that** the middle portion (30) may be positioned with its internal bore (34) onto an end piece (16) of the stem portion (10) and the stem portion (10) may be clamped in this bore (34) by means of a clamping element (20).

2. A shoulder endoprosthesis according to claim 1, **characterised in that** the stem portion (10) may be clamped in the bore (34) in the middle portion (30) by a cone clamp.

3. A shoulder endoprosthesis according to claim 2, **characterised in that** the proximal end (16) of the stem portion (10) is expandable and comprises a conical bore (12), in which the clamping element (20) engages.

4. A shoulder endoprosthesis according to claim 2, **characterised in that** the clamping element (20) is an expandable bush with a conical inner surface and the proximal end of the stem portion (10) comprises a conical outer surface (13), on which the clamping element (20) acts.

5. A shoulder endoprosthesis according to any one of claims 1 to 4, **characterised in that** the bore (34) in the middle portion (30) comprises an internal thread (35) and the clamping element (20) or the proximal end of the stem portion (10) has a corresponding external thread.

6. A shoulder endoprosthesis according to any one of claims 1 to 4, **characterised in that** the bore (34) in the middle portion (30) has a smooth inner surface and the clamping element (20) or the proximal end of the stem portion (10) has a smooth outer surface.

7. A shoulder endoprosthesis according to any one of claims 1 to 6, **characterised in that** the middle portion (30) has on its outer surface an element (36) for fixing bone fragments.

## Revendications

1. Endoprothèse d'épaule avec une tête d'articulation (40), avec un élément central (30) auquel la tête d'articulation (40) peut être fixée, et avec une tige (10) qui peut également être fixée à l'élément central (30), l'élément central (30) étant réglable par rapport à la tige (10) le long de l'axe de la tige (A) de ce dernier, l'élément central (30) étant réglable dans sa position de rotation autour de l'axe de la tige (A) et l'élément central (30) comportant un perçage (34), **caractérisée en ce que** l'élément central (30) avec son perçage intérieur (34) peut être emboîté sur une extrémité (16) de la tige (10) et **en ce que** la tige (10) peut être serrée dans ce perçage (34) à l'aide d'un élément de serrage (20).

2. Endoprothèse d'épaule selon la revendication 1, **caractérisée en ce que** la tige (10) peut être serrée dans le perçage (34) de l'élément central (30) à l'aide d'un dispositif de serrage à cône.

3. Endoprothèse d'épaule selon la revendication 2, **caractérisée en ce que** l'extrémité proximale (16) de la tige (10) est extensible et comporte un perçage conique (12) dans lequel l'élément de serrage (20) vient en prise.

4. Endoprothèse d'épaule selon la revendication 2, **caractérisée en ce que** l'élément de serrage (20) est une douille extensible avec une surface intérieure conique et **en ce que** l'extrémité proximale de la tige (10) comporte une surface extérieure conique (13) sur laquelle l'élément de serrage (20) vient en prise.

5. Endoprothèse d'épaule selon une des revendications 1 à 4, **caractérisée en ce que** le perçage (34) de l'élément central (30) comporte un filetage intérieur (35) et **en ce que** l'élément de serrage (20) et/ou l'extrémité proximale de la tige (10) ont un filetage extérieur correspondant.

6. Endoprothèse d'épaule selon une des revendications 1 à 4, **caractérisée en ce que** le perçage (34) de l'élément central (30) a une surface intérieure lisse et **en ce que** l'élément de serrage (20) et/ou l'extrémité proximale de la tige (10) ont une surface extérieure lisse.

7. Endoprothèse d'épaule selon une des revendications 1 à 6, **caractérisée en ce que** l'élément central (30) dispose sur la surface extérieure d'un élément (36) pour la fixation de fragments d'os brisés.
